# FASCICULE DE BREVET EUROPEEN

(11) **EP 3 019 179 B1**
(45) Date de publication et mention de la délivrance du brevet: **13.01.2021**
(21) Numéro de dépôt: 14739769.9
(22) Date de dépôt: 09.07.2014
(51) Int. Cl.: A61K 35/33, A61P 17/14, A61K 31/505, A61K 31/58, A61Q 7/00, A61K 8/98

(54) **UTILISATION DE FIBROBLASTES GINGIVAUX DANS LE TRAITEMENT DE L'ALOPECIE**
VERWENDUNG VON ZAHNFLEISCHFIBROBLASTEN BEI DER BEHANDLUNG VON ALOPEZIE
USE OF GINGIVAL FIBROBLASTS IN THE TREATMENT OF ALOPECIA

(30) Priorité: 09.07.2013 FR 1356740
(43) Date de publication de la demande: 18.05.2016
(73) Titulaire: Assistance Publique Hôpitaux de Paris, 75004 Paris (FR); Institut National de la Santé et de la Recherche Médicale (INSERM), 75013 Paris (FR); Institut de Radioprotection et de Sûreté Nucléaire, 92260 Fontenay aux Roses (FR); Etat Français - Ministère De La Défense Direction Centrale du Service de Santé des Armées, 92141 Clamart Cedex (FR); Université Paris Descartes, 75006 Paris (FR)
(72) Inventeur: LAFONT, Antoine, F-75007 Paris (FR); COULOMB, Bernard, F-91430 Igny (FR); LATAILLADE, Jean-Jacques, F-40300 Saint Lon les Mines (FR); LINARD, Christine, F-91130 Ris Orangis (FR)
(74) Mandataire: Vial, Lionel
(86) Numéro de dépôt international: PCT/EP2014/064772
(87) Numéro de publication internationale: WO 2015/004216

(56) Documents cités:
- EP-A1- 1 972 685
- EP-A2- 0 455 422
- WO-A1-2009/121761
- WO-A2-2008/017927
- FR-A1- 2 872 431
- SCHNEIDER M R ET AL: "The Hair Follicle as a Dynamic Miniorgan", CURRENT BIOLOGY, CURRENT SCIENCE, GB, vol. 19, no. 3, 10 février 2009 (2009-02-10), pages R132-R142, XP025916034, ISSN: 0960-9822, DOI: 10.1016/J.CUB.2008.12.005 [extrait le 2009-02-09]
- PAUS ET AL: "Therapeutic strategies for treating hair loss", DRUG DISCOVERY TODAY: THERAPEUTIC STRATEGIES, ELSEVIER, AMSTERDAM, NL, vol. 3, no. 1, 1 avril 2006 (2006-04-01), pages 101-110, XP005394597, ISSN: 1740-6773, DOI: 10.1016/J.DDSTR.2006.03.004
- EBISAWA K ET AL: "The Expression Profile of Anti-Aging Related Genes in Dermal and Gingival Fibroblasts", ANNALS OF NUTRITION AND METABOLISM: EUROPEAN JOURNAL OF NUTRITION, METABOLIC DISEASES AND DIETETICS, S. KARGER AG, SWITZERLAND, vol. 51, 1 novembre 2007 (2007-11-01), pages 409-415, XP002535647, ISSN: 0250-6807, DOI: 10.1159/000110786
- ROGERS N E ET AL: "Medical treatments for male and female pattern hair loss", JOURNAL OF THE AMERICAN ACADEMY OF DERMATOLOGY, C.V. MOSBY, ST. LOUIS, MO, US, vol. 59, no. 4, 1 octobre 2008 (2008-10-01), pages 547-566, XP025470517, ISSN: 0190-9622, DOI: 10.1016/J.JAAD.2008.07.001 [extrait le 2008-09-15]
- LEYDEN J ET AL: "Finasteride in the treatment of men with frontal male pattern hair loss", JOURNAL OF THE AMERICAN ACADEMY OF DERMATOLOGY, MOSBY, INC, US, vol. 40, no. 6, 1 juin 1999 (1999-06-01), pages 930-937, XP027523197, ISSN: 0190-9622 [extrait le 1999-06-01]

## Description

La présente invention concerne l'utilisation de fibroblastes gingivaux ou de produits dérivés pour limiter la chute des cheveux/poils et/ou promouvoir leur croissance. Plus particulièrement, l'invention concerne un produit dérivé de fibroblaste gingival pour son utilisation dans le traitement ou la prévention de l'alopécie, ainsi que dans la promotion de la croissance naturelle des cheveux et/ou la lutte contre la chute naturelle des cheveux.

La croissance des cheveux et leur renouvellement sont principalement déterminés par l'activité des follicules pileux. Chez l'être humain, les follicules pileux possèdent, comme chez la souris et les autres mammifères à fourrures, un cycle de renouvellement comportant les trois phases : anagène, catagène et télogène. La phase anagène, ou phase de croissance, dure plusieurs années et voit les cheveux s'allonger. Lui succède une phase catagène, ou phase de transition, qui dure environ 3 semaines, puis une phase télogène, ou phase de repos, à la fin de laquelle l'ancien cheveu tombe et laisse place à un nouveau follicule en phase anagène (Cotsarelis et al., Trends in Molecular Medicine, Vol. 7 No. 7, 2001). La chevelure se renouvelle donc en permanence et, sur les 100 000 à 150 000 cheveux que comporte une chevelure, la majorité est en phase de croissance (soit environ 85% en phase anagène, 2% étant en phase télogène et plus de 10% en phase de chute). En moyenne, l'être humain perd ainsi environ 50 à 150 cheveux par jour, cette chute « normale » prédominant généralement à la fin de l'été et au printemps (jusqu'à environ 175 cheveux perdus par jour). Une chute de plus de 100 à 150 cheveux par jour sera ainsi considérée comme pathologique si elle dure pendant une assez longue période pouvant aller jusqu'à deux mois.

Le terme alopécie désigne la perte partielle ou générale des cheveux. De nombreux facteurs peuvent être impliqués dans l'alopécie comme par exemple les facteurs génétiques, l'âge, le sexe, les maladies, le stress, les problèmes hormonaux, les effets secondaire de médicaments, les cicatrices. Il est possible de distinguer plusieurs formes d'alopécie :
- l'alopécie androgénétique héréditaire : elle est la plus fréquente. La chute précoce des cheveux survient chez des sujets prédisposés génétiquement et elle atteint en particulier les hommes. Elle se manifeste par une diminution du volume des cheveux, voire une calvitie, et touche 50 % des hommes âgés de plus de 50 ans (Yazdan P. Semin Cutan Med Surg 2012 ; 31 :258-266) ;
- l'alopécie post-ménopausique : il s'agit de la plus fréquente cause de la calvitie chez la femme. Chez la femme, la chute des cheveux est plus diffuse et étendue que chez l'homme. L'alopécie diffuse féminine est un désordre qui démarre souvent à la ménopause et qui concerne environ 40 % des femmes âgées de plus de 70 ans (Yazdan P. Semin Cutan Med Surg 2012 ; 31 :258-266). Le terme diffus illustre que, contrairement à l'homme, la perte des cheveux concerne la totalité du cuir chevelu, de manière homogène ;

- l'alopécie aiguë : elle peut être liée à un traitement par chimiothérapie, un stress, des carences alimentaires importantes, une carence en fer, des troubles hormonaux ;
- l'alopécie cicatricielle : elle peut être provoquée par des problèmes de peau (tumeur, brûlure, pelade), une irradiation aiguë, au lupus érythémateux ou des parasites (teigne, lichen) ;
- l'alopécie *areata*: elle semble être d'origine auto-immune et se caractérise par une atteinte en plaques plus ou moins larges et à un ou plusieurs endroits ; et
- l'alopécie congénitale : rare, elle correspond à une absence de racines ou à des anomalies du cheveu (mutations).

L'alopécie est essentiellement liée à une perturbation du renouvellement capillaire qui entraine, dans un premier temps, l'accélération de la fréquence des cycles aux dépens de la qualité des cheveux puis de leur quantité. Le phénomène le plus fréquent est une réduction du cycle de croissance (phase anagène) dû à un arrêt de la prolifération cellulaire. La conséquence est une induction prématurée de la phase catagène et un nombre plus important de follicules pileux en phase télogène et donc une chute plus importante des cheveux. Pour lutter contre l'alopécie, il est donc nécessaire de relancer le cycle pilaire, par exemple, en activant la phase anagène.

Les industries cosmétique et pharmaceutique recherchent depuis de nombreuses années des compositions permettant non seulement de supprimer ou de réduire l'alopécie, en particulier androgénétique, mais également de stimuler la croissance naturelle des cheveux et/ou de ralentir leur chute pour des raisons purement esthétiques, en particulier chez des individus non atteints d'alopécie souhaitant avoir néanmoins une chevelure plus épaisse et/ou plus longue.

Le traitement de l'alopécie ainsi que la promotion de la pousse naturelle des cheveux ont fait l'objet de nombreuses recherches, et plusieurs produits ou techniques sont disponibles.

La littérature fait état de nombreux composés destinés à traiter ou à prévenir l'alopécie et à restaurer ou promouvoir la croissance des cheveux ou des poils. Par exemple, le finastéride (Propecia®) est capable de stabiliser la chute des cheveux et, dans certains cas, de permettre une repousse plus ou moins importante. Néanmoins, ce composé présente de nombreux effets secondaires indésirables tels que baisse de la libido ou la dépression. Le minoxidil (Rogaine®) est, quant à lui, une autre composition commercialisée pour traiter la calvitie ; néanmoins, il semble que les cheveux obtenus grâce au produit tombent lors de l'arrêt de son application.

Des agents cosmétiques destinés à conférer du volume aux chevelures fines sont également commercialisés, en particulier dans des shampooings. Ceux-ci ne permettent néanmoins pas de stimuler la pousse des cheveux, et leur effet cesse également dès l'arrêt de leur utilisation.

Des procédés de micro-greffe ont également été développés. Cette technique consiste à prélever des greffons de cheveux sur la couronne (ou les cheveux éternels) et les greffer sur le haut de la tête. Néanmoins certains greffons peuvent tomber 2 à 4 semaines après la transplantation et cette technique nécessite plusieurs interventions pour être efficace.

Les cellules souches ont récemment reçu une attention particulière en raison de leur capacité à régénérer les tissus et les organes. Ainsi, une autre méthode possible serait d'introduire des cellules souches capables de se différencier en un follicule de cheveux afin de promouvoir la croissance des cheveux sur la peau humaine (US2008254006).

Ainsi, malgré les nombreux développements sur cette problématique, il reste un besoin pour de nouveaux traitements capables d'accélérer la croissance /ralentir la chute des cheveux et/ou des poils et d'augmenter la densité des follicules pileux, que ce soit dans le cadre d'une alopécie, ou d'une perte naturelle des cheveux et/ou poils. Ces besoins sont résolus par la présente invention décrite ci-après.

### Résumé de l'invention

La présente invention découle de la mise en évidence inattendue, par les inventeurs, que l'administration de fibroblastes gingivaux est capable de stimuler de façon significative la croissance des cheveux ou des poils.

Ainsi, l'invention concerne un produit dérivé de fibroblaste gingival pour son utilisation dans le traitement ou la prévention de l'alopécie.

Plus particulièrement, l'invention concerne un produit dérivé de fibroblaste gingival pour son utilisation dans le traitement ou la prévention de l'alopécie androgénétique héréditaire, l'alopécie post-ménopausique, l'alopécie aiguë ou l'alopécie areata.

L'invention concerne également un produit dérivé de fibroblaste gingival, pour son utilisation dans le traitement ou la prévention de l'alopécie, administré par voie intradermique ou topique.

Dans un autre aspect, la présente invention concerne l'utilisation cosmétique d'un produit dérivé de fibroblaste gingival pour limiter la chute des cheveux/poils et/ou promouvoir leur croissance et/ou augmenter la densité des follicules pileux. Plus particulièrement, cette utilisation permet de promouvoir la croissance des cheveux et augmenter la densité des follicules pileux. De manière préférée, cette utilisation cosmétique vise à lutter contre la chute naturelle des cheveux/poils et/ou à stimuler leur croissance naturelle et/ou leur densité naturelle.

Dans un aspect préféré, la présente invention concerne l'utilisation cosmétique d'un produit dérivé de fibroblaste gingival pour limiter la chute des cheveux/poils et/ou promouvoir leur croissance et/ou augmenter la densité des follicules pileux chez un individu présentant les conséquences d'une alopécie.

Dans un autre aspect, la présente invention concerne également un procédé pour limiter la chute cheveux/poils et/ou promouvoir leur croissance et/ou augmenter la densité des follicules pileux comprenant une étape d'administration d'un produit dérivé de fibroblaste gingival à un individu.

Plus particulièrement, ce procédé de traitement comprend ou consiste en les étapes suivantes :
∘ prélever des fibroblastes gingivaux d'un individu,
∘ éventuellement, cultiver les fibroblastes gingivaux,
∘ obtenir un produit dérivé de fibroblaste gingival, et
∘ administrer le produit dérivé de fibroblaste gingival.

Selon la présente invention le produit dérivé de fibroblaste gingival peut être sélectionné parmi i) des fibroblastes gingivaux, par exemple directement recueillis à partir du tissu gingival, ii) des fibroblastes gingivaux issus d'une culture, iii) un extrait de fibroblaste gingival et, iv) un milieu conditionné par des fibroblastes gingivaux.

La présente invention peut être utilisée en association avec des traitements connus de l'alopécie ou des molécules connues pour favoriser la croissance des cheveux/poils ou limiter leurs chutes comme par exemple le finastéride, le minoxidil ou bénéficier à un individu qui a subi ou va subir une microgreffe.

### Description des figures

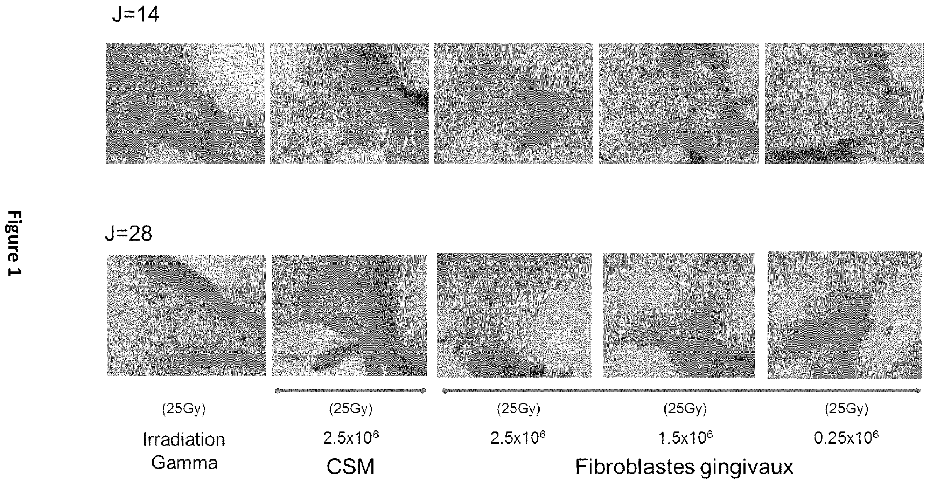

Figure 1. Efficacité supérieure des fibroblastes gingivaux humains par rapport aux cellules stromales mésenchymateuses (CSM) pour promouvoir la croissance des poils. L'observation des animaux 28 jours après l'irradiation gamma (25Gy) de la patte postérieure de souris NOD/SCID greffées le lendemain de l'irradiation montre que les 3 concentrations de fibroblastes gingivaux humains utilisées stimulent toutes la repousse du pelage. Les CSM de moelle osseuse humaine n'ont pas cette efficacité.

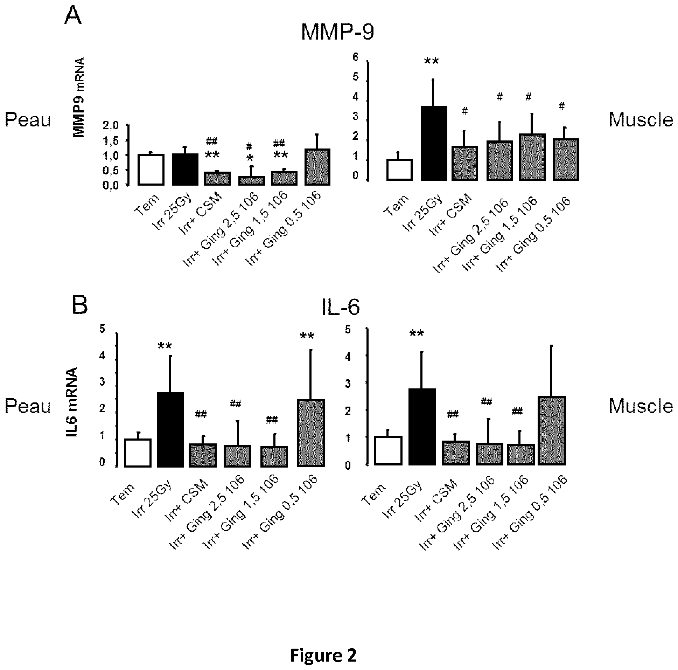

Figure 2. Effet similaire des fibroblastes gingivaux humains et des CSM sur le contrôle de l'inflammation et la régulation du remodelage de la matrice du derme. L'analyse des ARNm de la MMP-9 (A) et de l'IL-6 (B), 40 jours après l'irradiation (25Gy) dans la peau et les muscles des animaux greffés le lendemain de l'irradiation ne montre pas de différence d'expression significative entre les CSM (2,5x10⁶ cellules greffées) et les deux concentrations les plus élevées de fibroblastes gingivaux humains (2,5x10⁶ et 1,5x10⁶ cellules greffées). Les CSM et les fibroblastes gingivaux inhibent donc de façon comparable l'induction radio-induite de l'expression de la MMP-9 (Matrix Metalloproteinase 9; impliquée dans la dégradation de la matrice extracellulaire) et le l'IL-6 (Interleukine 6; Impliquée dans le processus inflammatoire).

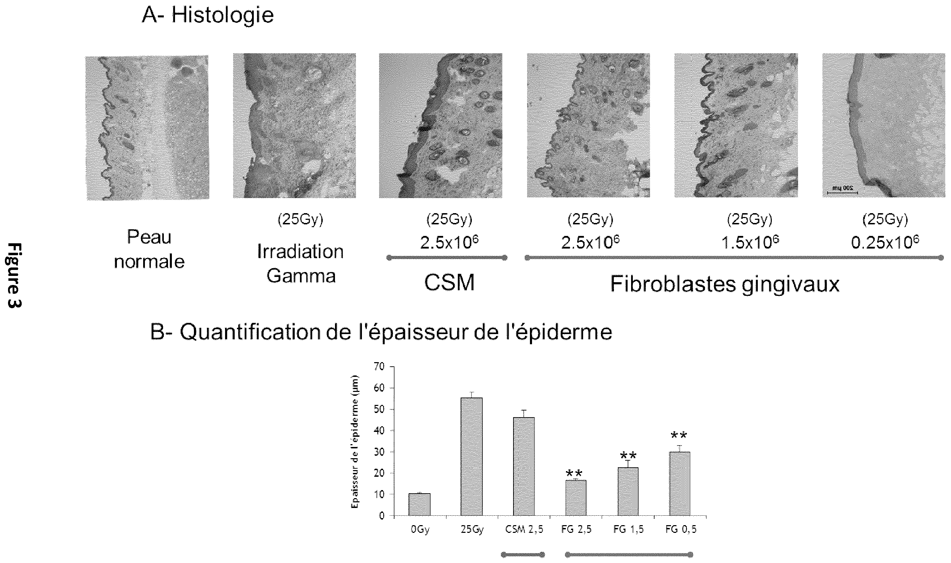

Figure 3. Effet supérieur des fibroblastes gingivaux humains par rapport au CSM sur la régulation de la différenciation épidermique. L'analyse histologique (A) 40 jours après l'irradiation (25Gy) dans la peau des animaux greffés le lendemain de l'irradiation montre une diminution de l'épaississement radio-induit de l'épiderme par les fibroblastes gingivaux humains. Ce qui n'est pas le cas pour les CSM. La quantification de l'épaisseur des épidermes (B) montre que cet effet régulateur de la différenciation épidermique par les fibroblastes gingivaux est dose-dépendant.

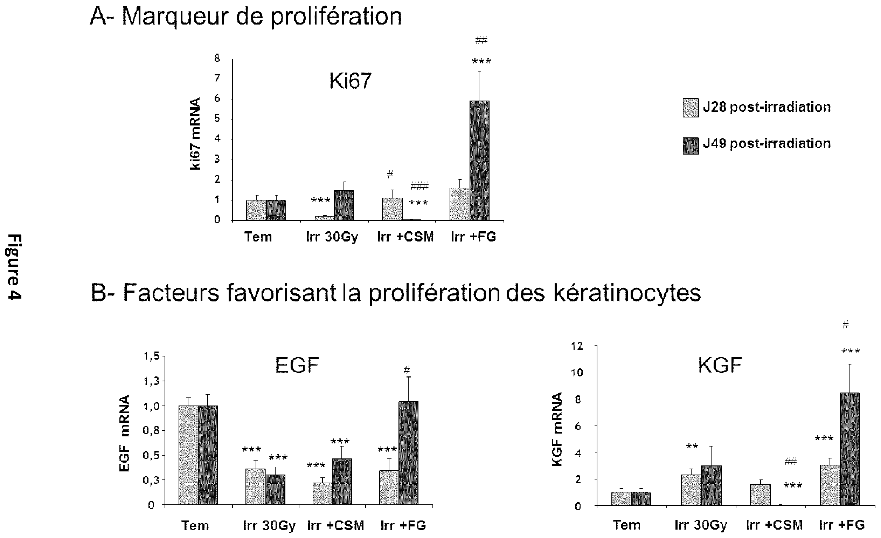

Figure 4. Efficacité supérieure des fibroblastes gingivaux humains par rapport aux CSM pour promouvoir la croissance épidermique. L'analyse des ARNm de facteurs considérés comme des marqueurs de prolifération (A; Ki67), ou favorisant la prolifération des kératinocytes (B; EGF & KGF), 49 jours après l'irradiation (30Gy) dans la peau des animaux greffés 21 jours après l'irradiation montre une stimulation de leur expression par les fibroblastes gingivaux humains (1,5x10⁶ cellules greffées). Cette stimulation n'existe pas pour les CSM (2,5x10⁶ cellules greffées). (A)- Ki67 est une protéine nucléaire nécessaire à la prolifération cellulaire. L'expression de son ARNm chute 1 semaine après l'irradiation gamma même si elle revient ensuite à une valeur proche de celle existant dans la peau non irradiée. Seule la greffe de fibroblastes gingivaux stimule son expression tardivement après l'irradiation (49 jours) témoignant d'une multiplication cellulaire nécessaire à la réorganisation de la zone lésée. (B)- Seuls les fibroblastes gingivaux humains permettent de retrouver un niveau d'expression de l'EGF (Epidermal growth factor) comparable à celui d'une peau non irradiée 49 jours après l'irradiation. Concernant le KGF (Keratinocyte growth factor ou FGF-7), seuls les fibroblastes gingivaux stimulent son expression encore 49 jours après l'irradiation. Ces deux facteurs sont importants pour permettre la croissance des kératinocytes participant non seulement à l'organisation de l'épiderme inter-folliculaire, mais aussi à la prolifération des kératinocytes des follicules pileux.

### Description détaillée de l'invention

La présente invention concerne l'utilisation d'un produit dérivé de fibroblaste gingival pour limiter la chute des cheveux/poils et/ou promouvoir leur croissance. L'invention concerne également un produit dérivé de fibroblaste gingival pour traiter ou prévenir l'alopécie, ainsi que leur utilisation pour promouvoir la croissance naturelle des cheveux et/ou ralentir la chute normale des cheveux chez des individus non atteints d'alopécie.

Par «cheveux/poils», on entend la chevelure, les poils, les sourcils, les cils et/ou le pelage. Préférentiellement, par «cheveux/poils», on entend les cheveux.

Ainsi, dans le contexte de la présente invention, le terme « sujet » ou « individu » se réfère préférablement à un mammifère, plus préférablement à un humain. L'individu peut également être un animal de compagnie tel que le chat, le chien, le furet, ou le lapin.

Par « alopécie », on entend la perte totale ou partielle des cheveux et/ou des poils par exemple liée à la réduction de la croissance capillaire et/ou à l'accélération de la chute des cheveux/poils. Ce terme inclut, mais ne se limite pas à l'alopécie androgénétique héréditaire, l'alopécie post-ménopausique, l'alopécie aiguë, l'alopécie cicatricielle, l'alopécie *areata,* et à l'alopécie congénitale. Les conséquences de l'alopécie sont une absence temporelle ou définitive et partielle ou totale de cheveux.

Par le terme « traitement » ou « traiter l'alopécie », on entend l'arrêt de l'alopécie, la réduction de l'alopécie et/ou l'atténuation de l'alopécie. Ainsi, « traiter l'alopécie » comprend limiter la chute des cheveux, promouvoir la croissance des cheveux, augmenter la densité des follicules pileux et/ou réguler les phases du cycle du follicule pileux. En d'autres termes, un tel traitement vise un sujet atteint d'alopécie.

Par le terme « prévenir » ou « prévention de l'alopécie », on entend diminuer le risque d'apparition de l'alopécie, ou de ralentir la progression de l'alopécie chez un mammifère qui est susceptible de développer une alopécie. En d'autres termes, ladite prévention vise un sujet atteint d'alopécie.

Par « chute naturelle des cheveux et/ou poils », ou « chute normale des cheveux et/ou poils », on entend ici une chute des cheveux et/ou poils habituellement observée chez un sujet présentant un cycle pileux physiologiquement normal (i.e. sain). De manière préférée, ladite chute est d'au plus une centaine de cheveux par jour. De manière encore préférée, ledit sujet n'est pas atteint d'alopécie.

Par « croissance naturelle des cheveux/poils », ou « croissance naturelle des cheveux/poils », on entend ici une croissance ou pousse des cheveux et/ou des poils habituellement observée chez un sujet présentant un cycle pileux physiologiquement normal (i.e. sain). De manière préférée, ledit sujet n'est pas atteint d'alopécie.

Par « densité naturelle des follicules pileux », on entend ici une densité des follicules pileux habituellement observée chez un sujet présentant un cycle pileux physiologiquement normal. Ainsi, chez l'être humain, selon le cycle pileux et son ratio de poils en phase anagène/télogène, ladite densité varie selon la zone du corps concernée, avec une moyenne de 200 à 300 cheveux/cm² sur le cuir chevelu, de 50 poils/cm² sur le visage, et de 10 poils/cm² sur le reste du corps. De manière préférée, ledit sujet n'est pas atteint d'alopécie.

Par le terme « limiter » ou « ralentir », on entend freiner, réduire, diminuer et/ou stopper. Ainsi, par « limiter ou ralentir la chute des cheveux et/ou poils », on entend freiner, réduire, diminuer, voire stopper, la chute des cheveux et/ou poils.

Par le terme « promouvoir », « stimuler » ou « augmenter », on entend accroitre, amplifier et/ou accélérer. Ainsi, par « promouvoir ou stimuler la croissance des cheveux et/ou poils », on entend augmenter, accroitre, amplifier et/ou accélérer, la croissance des cheveux et/ou poils.

Par « fibroblaste gingival » on entend au sens de l'invention des cellules mésenchymateuses retrouvées dans le tissu conjonctif mou de la gencive et capables de le remodeler et de le réparer grâce à leurs propriétés de synthèse, de migration, d'adhésion et de prolifération. Les fibroblastes gingivaux sont notamment décrits dans Gogly et al., (1997) Clin. Oral Invest. 1 :147-152; Gogly et al. (1998) Biochem. Pharmacol. 56:1447-1454; and Ejeil et al. (2003) J. Periodontol. 74:188-195.

Par « produit dérivé de fibroblaste gingival », on entend n'importe quel produit susceptible d'être obtenu à partir de fibroblastes gingivaux en eux-mêmes ou qui contient des sécrétions de fibroblastes gingivaux. On préfère ainsi que le produit dérivé de fibroblaste gingival selon l'invention soit sélectionné dans le groupe constitué par : i) des fibroblastes gingivaux, par exemple directement recueillis à partir du tissu gingival, ii) des fibroblastes gingivaux issus d'une culture, iii) un extrait de fibroblaste gingival et, iv) un milieu conditionné par des fibroblastes gingivaux.

Des cultures de fibroblastes gingivaux utilisables pour la mise en œuvre de la présente invention peuvent être obtenues par techniques classiques, qui sont connues en elles-mêmes de l'homme du métier (Barlovatz-Meimon et al., Culture de cellules animales, p. 898, ill. Paris : INSERM 2003).

L'extrait de fibroblaste gingival selon l'invention peut être obtenu par n'importe quelle méthode de fragmentation cellulaire connue de l'homme du métier. En particulier, l'extrait de fibroblaste gingival selon l'invention peut être un extrait membranaire, un extrait cytoplasmique ou un extrait nucléaire.

Le milieu conditionné par des fibroblastes gingivaux selon l'invention se réfère à n'importe quel milieu qui a été mis en contact avec des fibroblastes gingivaux. Le milieu peut être un milieu de culture liquide de cellule par exemple le milieu « Dulbecco's modified Eagle Médium » ou, de préférence, un milieu de culture sans sérum comme par exemple le milieu décrit dans EP1972685, US7951593 et CN102747033. De préférence, le milieu a été mis en contact avec les fibroblastes gingivaux pendant une durée suffisante pour que les fibroblastes gingivaux aient sécrété des composés dans le milieu. Par exemple, le milieu a été mis en contact avec les fibroblastes gingivaux pendant 1 à 7 jours, préférentiellement 1 jour et plus préférentiellement 1 jour dans un milieu sans sérum. Le milieu conditionné selon l'invention contient préférentiellement des molécules sécrétées par les cellules comme des constituants de la matrice extracellulaire, des facteurs de croissance et des cytokines. De façon préférée, le milieu conditionné ne contient pas de débris cellulaires.

De façon préférée, les produits dérivés de fibroblastes gingivaux selon l'invention comportent des cellules vivantes qui permettent d'avoir une action sur une longue durée. Ce mode de réalisation est particulièrement préféré dans le cadre la prévention ou du traitement de l'alopécie. La présence de cellules vivantes est cependant peu souhaitable en cosmétique. Ainsi, selon un autre mode de réalisation préféré de l'invention, les produits dérivés de fibroblastes gingivaux selon l'invention ne comportent pas de cellules vivantes, plus avantageusement dans le cadre de la lutte contre la chute naturelle des cheveux/poils, et/ou la promotion naturelle de leur croissance, et/ou l'augmentation de la densité naturelle des follicules pileux.

Lorsque les produits dérivés de fibroblaste gingival sont des fibroblastes gingivaux, par exemple directement recueillis à partir du tissu gingival (i), ceux-ci peuvent être obtenus par mise en suspension de fibroblastes gingivaux dans un milieu physiologiquement acceptable directement après prélèvement sur un individu ou après une étape de culture telle que décrite ci-dessus.

Comme cela est montré dans les exemples, cette invention repose sur les expériences menées par les inventeurs sur l'efficacité des fibroblastes gingivaux dans le traitement de l'alopécie et notamment des cellules stromales mésenchymateuses (CSM) de moelle osseuse et des fibroblastes gingivaux. Plus particulièrement, cette invention repose sur la découverte inattendue par les inventeurs de l'efficacité supérieure des fibroblastes gingivaux et de produits dérivés de fibroblaste gingival par rapport à des cellules stromales mésenchymateuses pour promouvoir la croissance des cheveux/poils, augmenter la densité des follicules pileux et réguler les phases du cycle du follicule pileux.

En effet, dans des essais *in vivo,* les inventeurs ont montré que l'administration d'une suspension de fibroblastes gingivaux humains au niveau d'une peau irradiée, lésion récente ou établie, entrainait en quelques semaines une repousse dense et uniforme des poils. De façon plus surprenante encore, la comparaison de l'efficacité des fibroblastes gingivaux humains à celles de CSM a montré que la longueur des poils a été significativement améliorée par les produits dérivés de fibroblastes gingivaux par rapport aux CSM.

Une étude approfondie des mécanismes impliqués dans cette repousse accélérée a montré que l'effet des CSM et des fibroblastes gingivaux ou des produits dérivés de fibroblastes gingivaux sur les marqueurs moléculaires du remodelage matriciel et de l'inflammation était similaire. En revanche, les fibroblastes gingivaux et les produits dérivés de fibroblastes gingivaux présentent un effet amélioré par rapport aux CSM sur la différenciation de l'épiderme et la régulation de son épaisseur. Cela induit une stimulation des follicules pileux, capables alors de générer une croissance plus rapide des cheveux/poils.

Les applications des produits dérivés de fibroblaste gingival selon l'invention visent à limiter la chute des cheveux/poils, et /ou à promouvoir leur croissance, et/ou à augmenter la densité des follicules pileux, plus particulièrement chez un sujet atteint d'alopécie, ou un sujet dont le cycle pileux est physiologiquement normal.

La présente invention concerne donc dans un premier aspect un produit dérivé de fibroblaste gingival pour son utilisation dans le traitement ou la prévention de l'alopécie. Plus particulièrement, la présente invention concerne un produit dérivé de fibroblaste gingival, en tant que médicament, pour le traitement ou la prévention de l'alopécie.

Selon un autre aspect, l'invention concerne une combinaison du produit dérivé de fibroblaste gingival tel que défini ci-dessus et d'au moins un agent anti-chute de cheveux pour une utilisation séparée, simultanée ou étalée dans le temps, dans la prévention ou le traitement de l'alopécie. Les agents anti-chute de cheveux particulièrement préférés selon l'invention sont le finastéride et le minoxidil.

Selon un autre aspect, la présente invention concerne une composition comprenant un produit dérivé de fibroblaste gingival pour son utilisation dans le traitement ou la prévention de l'alopécie. De manière préférée, ladite composition comprend en outre au moins un agent anti-chute de cheveux, tel que défini ci-dessus. La présente invention concerne également l'utilisation d'un produit dérivé de fibroblaste gingival pour la préparation d'une composition destinée au traitement ou la prévention de l'alopécie.

Tel qu'indiqué ci-dessus, la présente invention vise à limiter la chute des cheveux/poils et/ou promouvoir leur croissance et/ou augmenter la densité des follicules pileux. De façon préférée, la présente invention vise à promouvoir la croissance des cheveux/poils et à augmenter la densité des follicules pileux. Les produits dérivés de fibroblaste gingival selon l'invention peuvent être notamment utilisés dans un but purement esthétique.

Ainsi, un autre aspect de la présente invention concerne l'utilisation cosmétique d'un produit dérivé de fibroblaste gingival pour limiter la chute des cheveux/poils et/ou promouvoir leur croissance et/ou augmenter la densité des follicules pileux. De manière préférée, lesdites chute et/ou croissance et/ou densité des cheveux/poils sont dites naturelles selon les définitions données ci-dessus. De manière encore préférée, le sujet concerné n'est pas atteint d'alopécie.

La présente invention concerne également l'utilisation cosmétique d'un produit dérivé de fibroblaste gingival dans une composition pour limiter la chute des cheveux/poils et/ou promouvoir leur croissance, et/ou augmenter la densité des follicules pileux. De manière préférée, lesdites chute et/ou croissance et/ou densité des cheveux/poils sont dites naturelles selon les définitions données ci-dessus. De manière encore préférée, le sujet concerné n'est pas atteint d'alopécie.

De façon préférée, la présente invention concerne l'utilisation cosmétique d'un produit dérivé de fibroblaste gingival dans une composition pour promouvoir la croissance des cheveux/poils, et/ou augmenter la densité des follicules pileux. De manière préférée, lesdites croissance et/ou densité des cheveux/poils sont dites naturelles selon les définitions données ci-dessus. De manière encore préférée, le sujet concerné n'est pas atteint d'alopécie.

Dans le cadre d'une utilisation cosmétique d'un produit dérivé de fibroblaste gingival, ledit produit dérivé est de préférence iv) un milieu conditionné par des fibroblastes gingivaux. De manière préférée, pour cette utilisation, ledit milieu conditionné par des fibroblastes gingivaux ne contient pas de cellules vivantes.

Alternativement, de façon préférée, la présente invention concerne l'utilisation cosmétique d'un produit dérivé de fibroblaste gingival chez un individu présentant une absence partielle ou totale de cheveux/poils. Cette absence partielle ou totale de cheveux/poils peut correspondre aux conséquences d'une alopécie. L'absence partielle ou totale de cheveux/poils peut également correspondre aux conséquences d'une opération chirurgicale.

La présente invention concerne également un procédé pour limiter la chute des cheveux/poils et/ou promouvoir leur croissance et/ou augmenter la densité des follicules pileux comprenant une étape d'administration d'un produit dérivé de fibroblaste gingival à un individu. Dans le contexte de la présente invention, ledit individu peut être préférentiellement un sujet atteint d'alopécie, ou un sujet dont le cycle pileux est physiologiquement normal.

De façon préférée, l'invention porte sur un procédé pour promouvoir la croissance des cheveux comprenant une étape d'administration d'un produit dérivé de fibroblaste gingival à un individu.

De façon plus préférée, le procédé selon la présente invention comprend ou consiste en les étapes suivantes :
- prélever des fibroblastes gingivaux d'un individu,
- éventuellement, cultiver les fibroblastes gingivaux,
- obtenir un produit dérivé de fibroblaste gingival, et
- administrer le produit dérivé de fibroblaste gingival.

Dans un aspect particulièrement préféré, la présente invention concerne un procédé de traitement de l'alopécie comprenant une étape d'administration d'un produit dérivé de fibroblaste gingival à un individu. Les modes de réalisation préférés sont tels que définis ci-dessus.

Dans un autre aspect préféré, la présente invention concerne un procédé pour promouvoir la croissance naturelle des cheveux/poils, et/ou limiter la chute naturelle des cheveux/poils, et/ou augmenter la densité naturelle des follicules pileux comprenant une étape d'administration d'un produit dérivé de fibroblaste gingival à un individu. Les modes de réalisation préférés sont tels que définis ci-dessus.

La composition selon l'invention contient un produit dérivé de fibroblaste gingival (cellules, extrait et/ou milieu conditionné) en quantité suffisante pour obtenir l'effet recherché. L'effet recherché selon la présente invention est de limiter la chute des cheveux/poils, promouvoir leurs croissances, augmenter la densité des follicules pileux et/ou réguler les phases du cycle du follicule pileux.

Lorsque le produit dérivé de fibroblaste gingival comprend des cellules, celles-ci sont présentes à une concentration comprise entre 0,25 et 5 x 10⁶ cellules/mL, et plus préférentiellement entre 1,5 et 2,5 x 10⁶ cellules/mL.

Un des aspects surprenants de l'invention réside dans le fait que les produits dérivés de fibroblastes gingivaux sont bien plus efficaces pour la stimulation du follicule pileux que d'autres produits comprenant des cellules mésenchymateuses, notamment les CSM de moelle osseuse. Les CSM de moelle osseuse comprennent environ 10% de cellules progénitrices (Umbilical Cord Blood, ISBN: 978-981-283-329-7 ; Chap. 9 M Prat-Lepesant et al) alors que les fibroblastes gingivaux selon l'invention comprennent un maximum de 3 % de cellules progénitrices (Fournier BP et al., (2010) Tissue Eng Part A. 16(9) : 2891-9). Cet effet ne provient donc pas d'un nombre plus important de cellules progénitrices dans les produits de l'invention.

Dans un mode de réalisation spécifique, les produits dérivés de fibroblastes gingivaux peuvent en outre comprendre des cellules progénitrices de préférence moins de 20, 10 % ou de 5%.

Dans un mode de réalisation spécifique, les fibroblastes gingivaux peuvent par exemple être ceux décrits dans Fournier BP et al., (2010) Tissue Eng Part A. 16(9) : 2891-9.

Le mode d'administration dépend du produit dérivé et de la localisation de la zone traitée.

Selon un aspect de l'invention, le produit dérivé de fibroblaste gingival est présent dans une composition adaptée à une application intradermique ou topique pour le cuir chevelu et la peau, contenant un milieu physiologiquement acceptable.

Une composition « adaptée à une administration topique » peut notamment se présenter sous la forme d'une solution aqueuse, d'une émulsion de consistance liquide ou semi-liquide obtenue par dispersion d'une phase grasse dans une phase aqueuse ou inversement ou encore d'une microémulsion, de microcapsules, microparticules. Le pH d'une composition selon l'invention, lorsqu'elle comprend au moins une phase aqueuse, est de préférence compris entre 4 et 9, de préférence entre 4 et 7, avantageusement de 5 à 6.

Un « milieu physiologiquement acceptable » est, selon l'invention, un milieu cosmétiquement ou pharmaceutiquement acceptable compatible avec la peau, les muqueuses, les cheveux d'êtres humains et/ou le pelage de mammifères.

De préférence, l'individu selon l'invention est un mammifère, plus préférablement il s'agit d'un humain. L'individu peut également être un animal compagnie de tel que chat, chien, furet, lapin.

Les procédures pour prélever, cultiver et conserver les fibroblastes gingivaux sont bien connues de l'homme du métier et sont notamment décrites dans Naveau et al. (2006) J. Periodontol. 77 :238-47 et dans Gogly et al. (2007) Arterioscler. Thomb. Vase. Biol. 27 :1984-90.

Les fibroblastes gingivaux peuvent être hétérologues, c'est-à-dire obtenus à partir d'un individu d'une autre espèce. De préférence, les fibroblastes gingivaux utilisés selon l'invention sont allogéniques, c'est-à-dire obtenus à partir d'un autre individu de la même espèce. De façon encore plus préférée, les fibroblastes gingivaux utilisés selon l'invention sont autologues, c'est-à-dire qu'ils sont prélevés chez l'individu à qui le produit dérivé de fibroblaste doit être administré. Avantageusement, les fibroblastes gingivaux sont une source quasi illimitée de cellules autologues à administrer.

Avantageusement, les fibroblastes gingivaux sont aisément prélevables et cultivables. En outre, les fibroblastes gingivaux présentent une vitesse de croissance importante.

Avantageusement, les fibroblastes gingivaux sont plus efficaces pour stimuler la croissance des cheveux/poils que les autres fibroblastes ou cellules mésenchymateuses, notamment les CSM de moelle osseuse.

L'utilisation d'un produit dérivé de fibroblaste gingival selon l'invention est préférentiellement mise en œuvre sur les zones du cuir chevelu présentant une chute anormale des cheveux ou une absence de cheveux. L'identification de ces zones se fait aisément avant l'utilisation, ou l'application, du produit dérivé de fibroblaste gingival selon de l'invention.

L'administration à un individu tel que défini ci-dessus d'un produit dérivé de fibroblaste gingival selon l'invention, de préférence à proximité ou au niveau d'un site corporel à traiter, peut être effectuée par n'importe quelle méthode connue de l'homme du métier. Par exemple, en l'injectant à l'aide de microaiguilles dans le scalp.

La composition selon l'invention est une composition à application intradermique ou topique. Avantageusement, le produit dérivé de fibroblaste gingival est présent dans une composition adaptée à une application intradermique contenant un milieu physiologiquement acceptable.

On préfère que le produit dérivé de fibroblaste gingival ou la composition comprenant ledit produit soit administré(e) par injection en sous-cutané ou en intra-musculaire.

En outre, le produit dérivé de fibroblaste gingival peut être administré libre (e.g. en solution ou en suspension) ou associé à un support (e.g. encapsulé dans des microcapsules).

Selon l'invention, bien qu'efficace par lui-même, le produit dérivé de fibroblaste gingival peut être utilisé en association avec des traitements connus de l'alopécie ou des molécules connues pour favoriser la croissance des cheveux/poils ou limiter leurs chutes. Par exemple, le produit dérivé de fibroblaste gingival peut être utilisé en association avec du finastéride ou du minoxidil.

De façon préférée, selon la présente invention, le produit dérivé de fibroblaste gingival peut être utilisé avant, pendant ou après une micro-greffe. Les procédés de micro-greffe sont bien connus de l'homme du métier (M.J. Kristine Bunagan et al. 2013 Dermatologie Clinics, 31:1 p141-153) et ils pourraient avantageusement bénéficier de l'effet d'un produit dérivé de fibroblaste gingival sur la croissance des cheveux/poils et la densité des follicules pileux. En effet, l'administration d'un produit dérivé de fibroblaste gingival selon l'invention de par son effet sur la croissance des cheveux/poils et la densité des follicules pileux peut entrainer une efficacité supérieure de la microgreffe.

Le produit dérivé de fibroblaste gingival selon l'invention peut être utilisé par exemple de un mois avant à un mois après une microgreffe, préférentiellement de 15 jours avant à 15 jours après une microgreffe.

### Exemple

### Matériel et méthode

### Culture des cellules

Les fibroblastes gingivaux sont issus de donneurs sains. Après dissociation enzymatique des biopsies de gencives (collagénase, dispase), les fibroblastes gingivaux sont cultivés dans un milieu dépourvu de sérum, en présence de lysat plaquettaire (Doucet et al., J Cell Physiol. 2005; 205(2):228-36).

Les CSM sont recueillies à partir de la moelle osseuse de donneurs sains. Les CSM sont cultivées dans un milieu dépourvu de sérum, en présence de lysat plaquettaire (Doucet et al., J Cell Physiol. 2005; 205(2):228-36).

Après amplification, les fibroblastes gingivaux et/ou les CSM sont détachés de leur support de culture et mis en suspension dans du sérum physiologique avant d'être injectés en sous-cutané et intra-musculaire au niveau du site de la lésion.

### Modèle souris

Des souris NOD/SCID immunocompétentes (Possibilité de greffer des cellules humaines) ont été irradiées (25-30 Gy) au niveau d'une patte postérieure (Bensidhoum et al., J Soc Biol. 2005; 199(4): 337-41).

### Greffe

Selon l'expérience, le traitement a été effectué sur lésion aigue (1 jour après l'irradiation) ou établie (21 jours après l'irradiation). Chaque expérience comportait en général 5 animaux par condition.

Les cellules ont été injectées en sous-cutanée et intra-musculaire en plusieurs points dans la zone de la lésion (15 à 20 points). Le volume de la suspension cellulaire était de 200µL (Sérum physiologique). L'effet dose des fibroblastes gingivaux a été étudié en injectant 2,5x10⁶, ou 1,5x10⁶ ou 0,5x10⁶ cellules par souris. Un seule concentration a été retenue pour les CSM (2,5x10⁶) correspondant à la concentration utilisée en clinique humaine pour le traitement des irrradiés accidentels (Lataillade et al., Regen Med. 2007; 2(5): 785-94. Bey et al., Wound Repair Regen. 2010; 18(1): 50-8).

### Analyses

Le suivi des animaux a été fait sur 4 à 6 semaines (selon les expériences). Les animaux étaient sacrifiés en fin d'expérience pour analyse. Selon les expériences, un arrêt intermédiaire (1 semaine après la thérapie cellulaire) a été réalisé sur une partie des animaux.

Le suivi "macroscopique" consistait à une observation régulière des animaux avec prise de photographies de la zone lésée.

Après sacrifice des animaux, les tissus irradiés étaient prélevées. Une partie pour permettre l'analyse histologique, l'autre pour réaliser une extraction permettant l'analyse de l'expression des ARNm.

### Résultats et conclusions

L'observation "macroscopique" montre une amélioration de la lésion traitée par les fibroblastes gingivaux et les CSM, que le traitement ait été réalisé sur une lésion récente ou établie. Une repousse importante des poils est observée en présence de fibroblastes gingivaux (Figure 1). Les CSM n'induisent pas un tel effet sur la repousse du pelage.

L'expression des ARNm de la MMP-9 et de l'IL-6 ne diffère pas entre les fibroblastes gingivaux et les CSM, démontrant que les deux types cellulaires sont tous les deux efficaces pour stimuler le remodelage du derme et inhiber le processus inflammatoire (Figure 2). L'analyse histologique montre que les fibroblastes gingivaux humains régulent l'épaisseur de l'épiderme en inhibant l'augmentation radio-induite de l'épaisseur (Figure 3). Les CSM ne régulent pas cette différenciation de l'épiderme.

La stimulation de l'expression de Ki67 (marqueur d'une activité proliférative) seulement après greffe des fibroblastes gingivaux montre qu'ils contribuent fortement et de façon durable à la stimulation des divisions cellulaires nécessaires au remodelage des tissus (Figure 4-A). Les CSM n'ont qu'un effet discret et transitoire.

Les fibroblastes gingivaux stimulent également l'expression de l'EGF de façon durable, permettant de retrouver le niveau d'expression de la peau non irradiée. La stimulation de ce facteur dans la lésion contribue à la stimulation des divisions cellulaires nécessaire à la réorganisation des tissus. Les CSM ne le font pas de façon aussi intense et durable (Figure 4-B).

Enfin, l'action stimulante des fibroblastes gingivaux sur l'expression du KGF témoigne d'une action sur l'épiderme et plus particulièrement sur les kératinocytes; ce facteur n'ayant pas d'action stimulante sur la croissance des fibroblastes du derme (Rubin et al., Proc Natl Acad Sci USA. 1989; 86(3): 802-6) (Figure 4-B).

En conclusion, contrairement aux CSM issus de moelle osseuse humaine, les fibroblastes gingivaux humains stimulent fortement la repousse des poils. Cet effet est lié à une action plus large au niveau épidermique. Les fibroblastes gingivaux stimulent également de façon durable l'expression facteurs favorisant la prolifération des cellules épidermiques et plus particulièrement sur les kératinocytes.

## Revendications

1. Produit dérivé de fibroblaste gingival pour son utilisation dans le traitement ou la prévention de l'alopécie, **caractérisé en ce que** ledit produit dérivé de fibroblaste gingival est sélectionné parmi i) des fibroblastes gingivaux, par exemple directement recueillis à partir du tissu gingival, ii) des fibroblastes gingivaux issus d'une culture, iii) un extrait de fibroblaste gingival et iv) un milieu conditionné par des fibroblastes gingivaux.

2. Produit dérivé de fibroblaste gingival pour son utilisation selon la revendication 1, dans lequel l'alopécie est sélectionnée dans le groupe constitué de l'alopécie androgénétique héréditaire, l'alopécie post-ménopausique, l'alopécie aiguë, l'alopécie areata et l'alopécie congénitale.

3. Produit dérivé de fibroblaste gingival pour son utilisation selon la revendication 1 ou 2, **caractérisé en ce que** ledit fibroblaste gingival est d'origine hétérologue, allogénique ou autologue.

4. Produit dérivé de fibroblaste gingival pour son utilisation selon l'une quelconque des revendications 1 à 3, **caractérisé en ce que** ledit produit dérivé de fibroblaste gingival comprend des cellules vivantes.

5. Produit dérivé de fibroblaste gingival pour son utilisation selon l'une quelconque des revendications 1 à 4, **caractérisé en ce que** ledit produit dérivé de fibroblaste gingival est utilisé en association avec un traitement de l'alopécie sélectionné parmi le finastéride, le minoxidil ou une microgreffe.

6. Composition comprenant un produit dérivé de fibroblaste gingival tel que défini dans l'une quelconque des revendications 1 à 4, pour son utilisation dans le traitement ou la prévention de l'alopécie.

7. Composition selon la revendication 6, comprenant en outre au moins un agent anti-chute de cheveux.

8. Combinaison du produit dérivé de fibroblaste gingival tel que défini dans l'une quelconque des revendications 1 à 4 et d'au moins un agent anti-chute de cheveux pour une utilisation séparée, simultanée ou étalée dans le temps, dans la prévention ou le traitement de l'alopécie.

9. Composition selon la revendication 7 ou combinaison selon la revendication 8, **caractérisée en ce que** ledit agent est le finastéride ou le minoxidil.

10. Utilisation cosmétique d'un produit dérivé de fibroblaste gingival pour limiter la chute des cheveux/poils et/ou promouvoir leur croissance et/ou augmenter la densité des follicules pileux, **caractérisée en ce que** ledit produit dérivé de fibroblaste gingival est sélectionné parmi i) des fibroblastes gingivaux, par exemple directement recueillis à partir du tissu gingival, ii) des fibroblastes gingivaux issus d'une culture, iii) un extrait de fibroblaste gingival et iv) un milieu conditionné par des fibroblastes gingivaux.

11. Utilisation selon la revendication 10, **caractérisée en ce que** lesdites chute et/ou croissance et/ou densité sont naturelles.

12. Utilisation selon la revendication 10 ou 11, **caractérisée en ce que** ledit fibroblaste gingival est d'origine hétérologue, allogénique ou autologue.

13. Utilisation selon l'une quelconque des revendications 10 à 12, **caractérisée en ce que** ledit produit dérivé de fibroblaste gingival ne comprend pas de cellules vivantes.

## Patentansprüche

1. Aus Zahnfleisch-Fibroblasten gewonnenes Produkt zum Gebrauch in der Vorbeugung und Behandlung von Alopezie, **dadurch gekennzeichnet, dass** das genannte aus Zahnfleisch-Fibroblasten gewonnene Produkt aus der Gruppe gewählt ist, bestehend aus i) Zahnfleisch-Fibroblasten-Zellen, die beispielsweise direkt dem Zahnfleisch-Gewebe entnommen werden, ii) einer Zahnfleisch-Fibroblasten-Kultur entstammende Fibroblasten, iii) einem Zahnfleisch-Fibroblasten-Auszug und iv) einem mit Zahnfleisch-Fibroblasten behandelten Medium.

2. Aus Zahnfleisch-Fibroblasten gewonnenes Produkt zum Gebrauch nach Patentanspruch 1, wobei die Alopezie aus der Gruppe gewählt wird, bestehend aus erblicher androgenetischer Alopezie, Alopecia climacterica, akute Alopezie, Alopecia areata und kongenitale Alopezie.

3. Aus Zahnfleisch-Fibroblasten gewonnenes Produkt zum Gebrauch nach Patentanspruch 1 oder 2, **dadurch gekennzeichnet, dass** der genannte Zahnfleisch-Fibroblast heterologen, allogenen oder autologen Ursprungs ist.

4. Aus Zahnfleisch-Fibroblasten gewonnenes Produkt zum Gebrauch nach irgendeinem der Patentansprüche 1 bis 3, **dadurch gekennzeichnet, dass** das genannte aus Zahnfleisch-Fibroblasten gewonnene Produkt lebende Zellen enthält.

5. Aus Zahnfleisch-Fibroblasten gewonnenes Produkt zum Gebrauch nach irgendeinem der Patentansprüche 1 bis 4, **dadurch gekennzeichnet, dass** das genannte aus Zahnfleisch-Fibroblasten gewonnene Produkt in Verbindung mit einer Alopezie-Behandlung gebraucht wird, ausgewählt unter Finasterid, Minoxidil oder Follikelimplantation.

6. Zubereitung, ein aus Zahnfleisch-Fibroblasten gewonnenes Produkt nach irgendeinem der Patentansprüche 1 bis 4 enthaltend, zum Gebrauch in der Vorbeugung und Behandlung von Alopezie.

7. Zubereitung nach Patentanspruch 6, außerdem mindestens ein Mittel gegen Haarausfall enthaltend.

8. Kombination des aus Zahnfleisch-Fibroblasten gewonnenen Produktes nach irgendeinem der Patentansprüche 1 bis 4 mit mindestens einem Mittel gegen Haarausfall für getrennten, simultanen oder zeitlich verteilten Gebrauch in der Vorbeugung und Behandlung von Alopezie.

9. Zubereitung nach Patentanspruch 7 oder Kombination nach Patentanspruch 8, **dadurch gekennzeichnet, dass** das genannte Mittel Finasterid oder Minoxidil ist.

10. Kosmetischer Gebrauch eines aus Zahnfleisch-Fibroblasten gewonnenen Produktes zur Begrenzung von Haupt-/Körperhaarausfall und/oder Förderung des Haarwachstums und/oder Vergrößerung der Dichte der Haarfollikel, **dadurch gekennzeichnet, dass** das genannte aus Zahnfleisch-Fibroblasten gewonnene Produkt unter i) Zahnfleisch-Fibroblasten-Zellen, die beispielsweise direkt dem Zahnfleisch-Gewebe entnommen werden, ii) einer Zahnfleisch-Fibroblasten-Kultur entstammende Fibroblasten, iii) einem Zahnfleisch-Fibroblasten-Auszug und iv) einem mit Zahnfleisch-Fibroblasten behandelten Medium ausgewählt wird.

11. Gebrauch nach Patentanspruch 10, **dadurch gekennzeichnet, dass** der genannte Haarausfall und/oder das Haarwachstum und/oder die Dichte natürlich sind.

12. Gebrauch nach Patentanspruch 10 oder 11, **dadurch gekennzeichnet, dass** der genannte Zahnfleisch-Fibroblast heterologen, allogenen oder autologen Ursprungs ist.

13. Gebrauch nach irgendeinem der Patentansprüche 10 bis 12, **dadurch gekennzeichnet, dass** das genannte aus Zahnfleisch-Fibroblasten gewonnene Produkt keine lebenden Zellen enthält.

## Claims

1. A gingival fibroblast-derived product for use in the treatment or prevention of alopecia, **characterised in that** said gingival fibroblast-derived product is selected from i) gingival fibroblasts, for example directly collected from the gingival tissue, ii) gingival fibroblasts from a culture, iii) a gingival fibroblast extract and iv) a gingival fibroblast conditioned medium.

2. The gingival fibroblast-derived product for use according to claim 1, wherein alopecia is selected from the group consisting of hereditary androgenic alopecia, postmenopausal alopecia, acute alopecia, alopecia areata and congenital alopecia.

3. The gingival fibroblast-derived product for use according to claim 1 or 2, **characterised in that** said gingival fibroblast-derived product is heterologous, allogeneic or autologous.

4. The gingival fibroblast-derived product for use according to any one of claims 1 to 3, **characterised in that** said gingival fibroblast-derived product comprises living cells.

5. The gingival fibroblast-derived product for use according to any one of claims 1 to 4, **characterised in that** said gingival fibroblast-derived product is used in association with a known treatment for alopecia selected from finasteride, minoxidil or a micro-transplantation.

6. A composition comprising a gingival fibroblast-derived product as defined in any one of claims 1 to 4, for use in the treatment or prevention of alopecia.

7. A composition according to claim 6, further comprising at least one anti-hair loss agent.

8. A combination of the gingival fibroblast-derived product as defined in any one of claims 1 to 4 and of at least one anti-hair loss agent for a separate, simultaneous or sequential use, in the prevention or treatment of alopecia.

9. The composition according to claim 7 or the combination according to claim 8, wherein said agent is finasteride or minoxidil.

10. A cosmetic use of a gingival fibroblast-derived product to limit hair/body hair loss and/or to promote their growth and/or to increase the density of hair follicles, **characterised in that** said gingival fibroblast-derived product is selected from i) gingival fibroblasts, for example directly collected from the gingival tissue, ii) gingival fibroblasts from a culture, iii) a gingival fibroblast extract and iv) a gingival fibroblast conditioned medium.

11. The use according to claim 10, wherein said hair loss and/or hair growth and/or density are natural.

12. The use according to claim 10 or 11, **characterised in that** said gingival fibroblast is heterologous, autologous or allogeneic.

13. The use according to any one of claims 10 to 12, **characterised in that** said gingival fibroblast-derived product does not comprise living cells.
